# EUROPÄISCHE PATENTSCHRIFT

(11) **EP 2 315 759 B1**
(45) Veröffentlichungstag und Bekanntmachung des Hinweises auf die Patenterteilung: **20.11.2013**
(21) Anmeldenummer: 09777838.5
(22) Anmeldetag: 12.08.2009
(51) Int. Cl.: C07D 319/12

(54) **VERFAHREN ZUR LAGERUNG UND ZUM TRANSPORT VON ZYKLISCHEN DIESTERN**
METHOD FOR STORING AND TRANSPORTING CYCLICAL DIESTERS
PROCÉDÉ DE STOCKAGE ET DE TRANSPORT DE DI-ESTERS CYCLIQUES

(30) Priorität: 21.08.2008 EP 08014855
(43) Veröffentlichungstag der Anmeldung: 04.05.2011
(73) Patentinhaber: Uhde Inventa-Fischer GmbH, 13509 Berlin (DE)
(72) Erfinder: HAGEN, Rainer, 13465 Berlin (DE); MÜHLBAUER, Udo, 10119 Berlin (DE)
(74) Vertreter: Reitzle, Helmut
(86) Internationale Anmeldenummer: PCT/EP2009/005854
(87) Internationale Veröffentlichungsnummer: WO 2010/020370

(56) Entgegenhaltungen:
- WO-A-2008/065130
- WO-A-2008/065132
- DE-A1- 1 808 939
- US-A1- 2008 125 599

## Beschreibung

Die vorliegende Erfindung betrifft ein Verfahren zur Lagerung und/oder zum Transport von intramolekularen zyklischen Estern (Lactonen), insbesondere von Lactid.

Bei der Herstellung von biologisch abbaubaren Polyestern, wie beispielsweise Polylactiden, entsteht standardmäßig das Zwischenprodukt Dilactid. Dilactid kann als L,L-Dilactid, D,D-Dilactid, meso-Dilactid oder als Mischung aus zwei oder drei der genannten Isomeren anfallen. Die Eigenschaften der daraus durch Ringöffnungspolymerisation hergestellten Polymere hängt stark von der Reinheit oder dem Mischungsverhältnis der genannten Isomeren ab. Um den Markt mit PLA-Typen unterschiedlicher Eigenschaften und damit Verwendungszwecken flexibel bedienen zu können, ist es vorteilhaft, die Isomeren in geschmolzener Form zwischenzulagern oder zwischen weiter voneinander entfernten Anlagen zu transportieren. Allerdings ist bei der Lagerung zu beobachten, dass bei standardmäßiger Lagerung beispielsweise unter wasser- und sauerstoffhaltiger Normalatmosphäre Abbau- und Ringöffnungsreaktionen des Dilactids auftreten können, so dass das Dilactid durch die entstehenden Abbauprodukte verunreinigt wird und für die Ringöffnungspolymerisationsreaktion zur Herstellung des Polylactids aufgrund der hohen einzuhaltenden Reinheitsstandards nicht mehr geeignet ist.

Derartige Herstellungsverfahren sind beispielsweise aus US 2008/0125599 A1, DE 1 808 939 oder der WO 2008/065132 A1 bekannt.

Ferner betrifft die WO 2008/065132 A1 stabile Lactidpartikel.

Aus der Literatur ist bekannt, dass die Lagerung von Dilactid in geschmolzenem Zustand unbrauchbar ist (NL 2000454), während für andere Stoffe, wie z.B. epsilon-Caprolactam, diese Lagerungsform als bevorzugt beschrieben ist (DE 101 00 752).

Somit ist es Aufgabe der vorliegenden Erfindung, ein Verfahren zur Lagerung und dem Transport von geschmolzenen zyklischen Diestern, insbesondere Dilactid, anzugeben, bei dem Abbaureaktionen zum größten Teil vermieden werden.

Diese Aufgabe wird bezüglich des Verfahrens zur Lagerung und/oder dem Transport eines zyklischen Diesters mit den Merkmalen des Patentanspruchs 1 gelöst. Die abhängigen Ansprüche stellen dabei vorteilhafte Weiterbildungen dar.

Erfindungsgemäß wird ein Verfahren zur Lagerung und/oder zum Transport eines cyclischen Diesters der allgemeinen Formel I bereitgestellt, 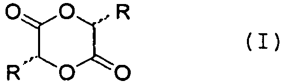 wobei R ausgewählt ist aus Wasserstoff oder linearen oder verzweigten aliphatischen Resten mit 1 bis 6 Kohlenstoffatomen, bei dem der Diester der allgemeinen Formel I nach der Herstellung unterbrechungslos direkt in flüssigem Aggregatszustand in ein inertisiertes Lagergefäß überführt und zumindest zeitweise bei 40 bis 150 °C im Lagergefäß gelagert und/oder transportiert wird. Die Lagerung und/oder der Transport erfolgt dabei bei einem Druck von 0,1 bar bis 10 bar, wobei der Wassergehalt des eingesetzten Diesters max. 100 ppm, bezogen auf das Gewicht, beträgt.

Überraschenderweise konnte beobachtet werden, dass gemäß dem erfindungsgemäßen Verfahren auch eine längere, mehrwöchige Lager- bzw. Transportzeit möglich ist, wobei die ursprünglichen Eigenschaften des eingesetzten Dilactids hinsichtlich des Säuregehaltes (Carboxylendgruppen-Konzentration) bzw. der diastereomeren Reinheit im Wesentlichen beibehalten werden können. Erfindungsgemäß wird somit Dilactid im flüssigen Aggregatszustand und in geschlossenen Behältern gelagert oder transportiert.

Die flüssige Phase wird im Allgemeinen auch als "Schmelze" bezeichnet. Sie kann beim erfindungsgemäßen Verfahren auch mit Feststoffpartikeln, wie etwa kristallinem Diester der Formel I, durchsetzt sein. Des Weiteren können Feststoffpartikel, wie etwa kristalliner Diester der Formel I, beispielsweise als Bodensatz vorliegen. Beim erfindungsgemäßen Verfahren hält man bevorzugt den überwiegenden Teil des zu lagernden bzw. zu transportierenden Diesters der Formel I in der flüssigen Phase. Besonders bevorzugt hält man über 90 Gew.-%, ganz besonders bevorzugt über 99 Gew.-% und insbesondere den gesamten Diester der Formel I in der flüssigen Phase. Falls man teilweises oder vollständiges Einfrieren der Schmelze im Behälter zulässt, kommt es darauf an, dass dies ohne Zutritt von O₂ und H₂O d.h. insbesondere unter Ausschluss der Umgebungsluft geschieht. Das gilt auch für das Wiederaufschmelzen des eingefrorenen Behälterinhalts. Wenn man diese Bedingungen einhält, kann der Abbau vermieden werden.

Um das Produkt in flüssiger Phase zu halten, wendet man im Allgemeinen eine Temperatur an, die dem Schmelzpunkt entspricht oder oberhalb des Schmelzpunktes liegt. Erfindungsgemäß beträgt die Lagertemperatur je nach eingesetztem Stoff dabei zwischen 40 °C bis 150 °C, wobei bevorzugte Temperaturbereiche zwischen 40 °C bis 130 °C betragen. Reines L-Dilactid bzw. dessen Enantiomer D-Dilactid, weist einen Schmelzpunkt von 98 °C auf. Die Schmelzepunkte von Gemischen aus den drei genannten Enantiomeren können zwischen 40°C und 125°C liegen. Demzufolge ist es besonders bevorzugt, wenn die Lagerung bzw. der Transport des Diesters, der besonders bevorzugt Dilactid sein kann, je nach Schmelzpunkt des eingesetzten Reinstoffes bzw. des eingesetzten Gemisches aus verschiedenen Enantiomeren bzw. diastereomeren Dilactiden 20 °C über dem Schmelzpunkt des jeweilig eingesetzten Stoffes bzw. Stoffgemisches liegt.

Die Zeitdauer für die Lagerung und den Transport beträgt beim erfindungsgemäßen Verfahren im Allgemeinen wenige Stunden bis mehrere Wochen. Sie kann gegebenenfalls auch kürzer oder länger sein. Besonders beträgt die Zeitdauer mindestens drei Tage, besonders bevorzugt mindestens fünf Tage und ganz besonders bevorzugt mindestens sieben Tage.

Besonders vorteilhaft beim Verfahren ist, dass kein Wechsel des Aggregatzustands des eingesetzten Diesters erfolgt, so dass der Diester unmittelbar aus dem Herstellungsprozess in flüssiger Form entnommen und dem Lagergefäß zugeführt werden kann. Besonders vorteilhaft ist hierbei, dass eine Auskristallisation des Produktes in Zuführungen oder Leitungen der Prozessapparaturen vollständig unterbleibt. Bevorzugt wird der Diester unmittelbar dem Herstellungsprozess bei einer Temperatur von 90 °C bis 160 °C in flüssigem Aggregatzustand entnommen und in das Lagergefäß überführt.

Der beim erfindungsgemäßen Verfahren eingesetzte Diester der Formel I besitzt im Allgemeinen eine bevorzugte stoffliche Reinheit von über 98 Gew.-%, weiter bevorzugt von über 99 Gew.-% und besonders bevorzugt von über 99,5 Gew.-%. Bei dem auf 100 Gew.-% fehlenden Anteil handelt es sich z.B. um Verunreinigungen, wie sie beispielsweise bei der Herstellung des Produkts oder beispielsweise durch Zersetzung, Ringöffnung, Oligomerisierung oder Polymerisierung des Diesters entstehen. Ein Maß für die Reinheit des Diesters der Formel I kann ebenso durch die Carboxylendgruppen-Konzentration angegeben werden. Für die Säureendgruppen-Konzentration im eingesetzten Diesters der Formel I sind dabei insbesondere zum Ester korrespondierende alpha-Hydroxycarbonsäure der Formel II 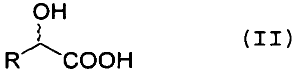 und/oder korrespondierenden Oligomeren der alpha-Hydroxycarbonsäure der Formel III, 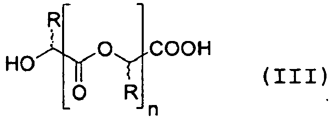 wobei n = 1 bis 10 ist und R je wie oben stehend definiert ist, verantwortlich. Der Diester wird dabei bevorzugt in einer Reinheit eingesetzt, dass die Carboxylendgruppen-Konzentration der Formel I maximal 20 mmol/kg, bevorzugt maximal 10 mmol/kg, besonders bevorzugt maximal 5 mmol/kg, insbesondere maximal 2 mmol/kg beträgt.

Beim erfindungsgemäßen Verfahren kann je nach eingesetzter Reinheit des Diesters beobachtet werden, dass die Carboxylendgruppen-Konzentration im Wesentlichen konstant bleibt, was mit der Tatsache gleichgesetzt werden kann, dass der Diester der Formel I unter den erfindungsgemäßen Lagerbedingungen nahezu nicht hydrolysiert bzw. oligo- oder polymerisiert wird.

Ein weiteres Qualitätskriterium des eingesetzten Diesters, mit dem sich ausgezeichnete Ergebnisse bei der Lagerung ergeben, ist der Wassergehalt. Dieser muss, bezogen auf den eingesetzten Diester, unterhalb 100 ppm (als Gewichtsanteile), bevorzugt unterhalb 50 ppm, insbesondere maximal 20 ppm betragen.

Das Lagergefäß weist dabei bevorzugt eine Inertgasatmosphäre auf, wobei das Inertgas so zu verstehen ist, dass es keine chemische Reaktion mit dem eingesetzten Diester eingehen kann. In der Regel können hierzu dem Fachmann allgemein bekannte Inertgase, wie beispielsweise Stickstoff und/oder Argon eingesetzt werden. Bevorzugt sind die dabei eingesetzten Gase im Wesentlichen frei von Sauerstoff und Wasser, weisen also lediglich einen vernachlässigbar geringen Sauerstoffpartialdruck bzw. Wassergehalt auf.

Der erfindungsgemäße Lagerdruck liegt zwischen 0,1 und 10 bar, wobei Drücke zwischen dem Umgebungsdruck und mäßigem Überdruck, also 1 bar bis 5 bar, bevorzugt sind.

Als bevorzugter Diester der oben stehenden Formel I wird insbesondere Dilactid eingesetzt, wobei es erfindungsgemäß unwesentlich ist, ob hier die D-, L- oder die meso-Form des Lactids zum Einsatz kommt. Ebenso lässt sich das Verfahren auch auf beliebige Mischungen der zuvor genannten verschiedenen enantio- bzw. diastereomeren Lactide anwenden. Insbesondere eignet sich das erfindungsgemäße Verfahren zur strukturstabilen Lagerung von reinem meso-Dilactid oder Mischungen aus verschiedenen Lactid-Formen mit hohem meso-Gehalt, also beispielsweise einem meso-Lactidgehalt von mehr als 60 Mol-%.

Für die Lagerung und den Transport setzt man im Allgemeinen dicht verschließbare Behälter ein. Ihre Größe ist für das erfindungsgemäße Verfahren im Allgemeinen unerheblich. In der Regel setzt man Behälter im dm³- und m³-Bereich ein. Auch die geometrische Form der Behälter ist für das erfindungsgemäße Verfahren im Allgemeinen unerheblich. Um Wärmeverluste zu minimieren, sollen Behälter ein niedriges Verhältnis von Oberfläche zu Volumen haben.Bevorzugte Beispiele sind: (i) im Wesentlichen kugelförmige Behälter (z.B. sog. Kugeltanks) und (ii) im Wesentlichen zylinderförmige Behälter (z.B. Flaschen, Fässer, sog. Zylindertanks oder Kesselwagen).

Die Wandung des Behälters muss gegenüber dem Ester der Formel I chemisch inert und gegenüber Gasen und Dämpfen, insbesondere Sauerstoff und Wasserdampf, undurchlässig sein. Geeignete Materialien sind beispielsweise Stahl, Edelstahl, Aluminium oder innenlackierte Metallgebinde. Bei Innenlackierung oder Beschichtung ist darauf zu achten, dass diese mit dem zyklischen Diester nicht quellen oder gelöst werden. Wichtig ist ebenso, dass Sauerstoff und Wasser weder ad- noch absorbiert werden. Geeignete Beschichtungsmaterialien sind z.B. PTFE oder PFA.

Bevorzugt lagert und transportiert man beim erfindungsgemäßen Verfahren den Diester der Formel I in einem wärmeisolierten Behälter. Darunter sind Behälter zu verstehen, welche an der Außenseite eine wärmedämmende Schicht aufweisen. Als geeignete wärmedämmende Schicht sind beispielsweise Zwischenschichten aus Vakuum, aus einem gering wärmeleitfähigen Gas, oder aus festen Dämmmaterialien, wie etwa geschäumtem Polystyrol (z.B. Styropor^{®}), Glas- oder Mineralwolle oder eine Kombination dieser Schichten zu nennen.

Bei der Lagerung und dem Transport des Diesters der Formel I in einem wärmeisolierten Behälter kann sich der Diester der Formel I (i) direkt ohne weitere Behälter oder (ii) verpackt in weiteren Behältern befinden. Als geeignete Beispiele zu (i) seien wärmeisolierte Tanks (z.B. Kugel-, Zylinder- oder Kesseltanks) oder wärmeisolierte Fässer genannt. Als geeignete Beispiele zu (ii) seien wärmeisolierte Container (z.B. Iso-Container), welche den Diester der Formel I in Form von weiteren Behältern (z.B. Fässer oder Flaschen) enthalten, genannt.

Besonders bevorzugt lagert und transportiert man beim erfindungsgemäßen Verfahren den Diester der Formel I in einem wärmeisolierten und beheizbaren Behälter. Darunter sind Behälter zu verstehen, welche neben der oben genannten Wärmeisolierung eine Heizung enthalten. Bei wärmeisolierten Behältern, in denen sich der Diester der Formel I (i) direkt ohne weitere Behälter befindet, kann sich die Heizung beispielsweise in Form von Heizelementen direkt im zu lagernden oder zu transportierenden Diester der Formel I befinden. Eine andere Möglichkeit besteht beispielsweise darin, dass sich die Heizung zwischen der Wärmeisolierung und der innen liegenden Behälterwand befindet. Als Beispiele hierzu seien mit einem Heizmantel versehene Fässer oder Tanks genannt. Bei wärmeisolierten Behältern, in denen sich der Diester der Formel I (ii) verpackt in weiteren Behältern befindet, befindet sich die Heizung üblicherweise im Inneren dieser Behälter.

In einer bevorzugten Ausführungsform des erfindungsgemäßen Verfahrens werden 220 L-Fässer in einem beheizbaren Iso-Container gelagert beziehungsweise transportiert.

Die Behälter können isoliert und beheizbar sein, entweder durch einen Doppelmantel oder durch eine innen liegende Heizschlange oder beides. Der Transport kann in Tanklastwagen auf der Straße oder in Kesselwagen auf der Schiene erfolgen. Die Größe und Form der Behälter ist aber nicht entscheidend.

Kleinere, unbeheizte Behälter, z.B. 220-1-Fässer, können in beheizbaren Containern gelagert und transportiert werden oder aber Transport und Lagerung erfolgen ohne Beheizung, mit der Folge, dass bei längerer Transport- und Lagerzeit der Inhalt einfriert. Dies ist für die Qualität des Produkts unerheblich, solange die Fässer dicht verschlossen bleiben. Um den Inhalt nach Lagerung und Transport wieder gebrauchsfähig zu machen, können die Fässer in eine mit temperierter Warmluft beheizte Kammer bis zum vollständigen Aufschmelzen des Inhalts eingesetzt werden. Dabei ist zu beachten, dass der Inhalt der Fässer den Schmelzpunkt um nicht mehr als 20 °C überschreitet. Die Produkttemperatur an der beheizten Wand sollte nicht über 150 °C betragen; jeder Luftzutritt muss auch während des Aufschmelzvorgangs ausgeschlossen sein.

Bei kurzen Transport- und Lagerzeiten (< 7 Tage) und großen Behältern (ab ungefähr 20 m³) kann auf eine permanente Beheizung verzichtet werden.

Eine gute Isolierung des Behälters reicht aus (abhängig von Region und Klima), um ein Einfrieren des Inhalts zu verhindern. Eventuell fest gewordene Teile des Inhalts werden durch Umpumpen der Flüssigkeit an der Entladestation wieder aufgeschmolzen.

Das erfindungsgemäße Verfahren zur Lagerung und zum Transport von Diester der Formel I ist besonders überraschend, da nach allgemeinem Fachwissen gerade in der flüssigen und somit diffusionsmobilen Phase eine wesentlich größere Reaktivität zu erwarten ist.

Das bevorzugte erfindungsgemäße Verfahren, bei dem man den Diester der Formel I bei der Schmelztemperatur des Diesters der Formel I oder darüber hält, ist insbesondere überraschend, da nach allgemeinem Fachwissen die Reaktivität in der Regel mit der Temperatur steigt.

Entscheidend für die Stabilität des Produkt ist jedoch das Vermeiden des Kontakts mit wasser- und sauerstoffhaltigen Medien und nicht eine möglichst niedrige Lager- und Transporttemperatur. Diese Erkenntnis kann nicht aus dem allgemeinen Fachwissen abgeleitet werden. Hieraus ergibt sich, dass Lagerung und Transport in flüssigem Zustand erfolgen sollen, da dabei und beim vorherigen Befüllen und anschließenden Entleeren der Lager- und Transportbehälter der Kontakt mit Wasser und Sauerstoff minimiert werden kann.

Das erfindungsgemäße Verfahren ermöglicht die Lagerung und den Transport von Diester der Formel I, wobei das Verfahren auch nach längerer, mehrwöchiger Lager- und Transportzeit nur zu einer geringen chemischen Veränderung des Produkts führt.

Die vorliegende Erfindung wird anhand der nachfolgenden Beispiele weiter verdeutlicht, ohne die Erfindung auf die nachfolgenden Parameter, insbesondere die speziellen Verhältnisse der einzelnen Lactide zueinander, zu beschränken.

### Beispiele

In einer Pilotanlage wurden durch Rektifikation von Rohlactid mit einem Gehalt an L-Dilactid von 12,2 % aus der Depolymerisation eines Milchsäure-Präpolymers zwei Dilactid-Fraktionen (Fraktion A und B) hergestellt. Fraktion A' wurde in einer weiteren Rektifikationskolonne gereinigt. Das gereinigte Produkt wird als Seitenstrom abgezweigt (Fraktion C). Die Fraktionen wurden während eines kontinuierlichen Betriebs der Rektifikationskolonnen bei einer Temperatur von 145 °C (Fraktion A), 152 °C (Fraktion B) und 148 °C (Fraktion C) entnommen und jeweils in ein 10 ml Headspace-Fläschchen, welches vorher 1 Stunde bei 110 °C getrocknet und anschließend 20 Minuten mit Argon gespült wurde, gefüllt. Gleichzeitig wurde jeweils eine Probe zur Analyse auf Carboxylgruppenkonzentration und meso-Diläctid-Gehalt entnommen.

### Beispiel 1

Ein mit Fraktion B gefülltes Fläschchen wurde mit Septum aus PTFE-beschichtetem Butylgummi sowie mit den üblichen Dichtscheiben und Bördelkappen verschlossen und für 24 h in einen auf 110 °C vorgeheizten Ofen gestellt. Die COOH-Konzentration betrug vorher und nachher jeweils 2 mmol/kg. Der meso-Dilactid-Gehalt betrug vor dem Versuch 4,1 % und nachher 3,9 %, wobei die Differenz zu 100 % aus L-Dilactid bestand.

### Beispiel 2

Das Vorgehen aus Beispiel 1 wurde wiederholt, nur dass dieses Mal das Fläschchen nicht verschlossen wurde. Nach 24 Stunden im Ofen stieg die COOH-Konzentration von 2 mmol/kg auf 69 mmol/kg an. Der meso-Dilactid-Gehalt betrug vorher 4,1 % und nachher 3,5 %.

Die Beispiele 1 und 2 zeigen, dass Quälitätsverluste während der flüssigen Lagerung nur unter Ausschluss von Wasser und Sauerstoff zu verhindern sind.

### Beispiel 3

Das Vorgehen aus Beispiel 1 wurde wiederholt. Dieses Mal wurde jeweils ein Fläschchen der Fraktionen A, B und C für drei Tage in einen auf 110 °C geheizten Ofen gestellt. Der Inhalt der Fläschchen war dabei zu jeder Zeit flüssig. Der Carboxylendgruppengehalt wurde mittels Titration und der meso-Dilactid-Anteil mit HPLC gemessen.

Die Ergebnisse sind in folgender Tabelle dargestellt:

| **COOH** | **vorher** | **nachher** |
|---|---|---|
| Fraktion A | 217 mmol/kg | 298 mmol/kg |
| Fraktion B | 18 mmol/kg | 18 mmol/kg |
| Fraktion C | 25 mmol/kg | 31 mmol/kg |

| **Meso-Dilactid-Gehalt** | **vorher** | **nachher** * |
|---|---|---|
| Fraktion A | 78,5 % | 75,9 % |
| Fraktion B | 5,9 % | 4,9 % |
| Fraktion C | 95,2 % | 94,1 % |

| | | |
|---|---|---|
| * Bei der Differenz handelt es sich zu 100 % um L-Dilactid. | | |

### Beispiel 4

Der Versuch aus Beispiel 3 wurde wiederholt, wobei die Reinheit von Fraktion B und Fraktion C bezüglich der Carboxylendgruppen größer war.

| **COOH** | **vorher** | **nachher** |
|---|---|---|
| Fraktion A | 197 mmol/kg | 278 mmol/kg |
| Fraktion B | 2 mmol/kg | 2 mmol/kg |
| Fraktion C | 1 mmol/kg | 1 mmol/kg |

| **Meso-Dilactid-Gehalt** | **vorher** | **nachher** * |
|---|---|---|
| Fraktion A | 68,7 % | 64,5 % |
| Fraktion B | 4,1 % | 4,1 % |
| Fraktion C | 92,0 % | 92,1 %. |

| | | |
|---|---|---|
| * Bei der Differenz handelt es sich zu 100 % um L-Dilactid. | | |

Insbesondere bei hohen meso-Dilactid- und COOH-Konzentrationen ist zu erkennen, dass meso-Dilactid leichter gespalten wird als L,L-Dilactid. Während die COOH-Konzentration zunimmt, reduziert sich der Anteil an meso-Dilactid. Man erkennt auch, dass bei einem hohen L,L-Dilactid-Gehalt das Dilactid selbst bei einer COOH-Konzentration von 18 mmol/kg noch stabil ist, während es eine meso-Dilactid-reiche Fraktion mit 25 mmol/kg COOH nicht ist (Beispiel 3). Beispiel 4 zeigt, dass bei COOH-Konzentrationen unter 2 mmol/kg auch meso-Dilactid die nötige Stabilität besitzt.

## Patentansprüche

1. Verfahren zur Lagerung und/oder Transport eines cyclischen Diesters der allgemeinen Formel I 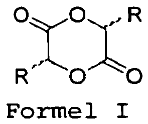 wobei R ausgewählt ist aus Wasserstoff oder linearen oder verzweigten aliphatischen Resten mit 1 bis 6 Kohlenstoffatomen, **dadurch gekennzeichnet, dass** der Diester der allgemeinen Formel I nach der Herstellung unterbrechungslos direkt in flüssigem Aggregatszustand in ein inertisiertes Lagergefäß überführt und zumindest zeitweise bei 40 bis 150 °C im Lagergefäß gelagert und/oder transportiert wird, wobei die Lagerung und/oder der Transport bei einem Druck von 0,1 bar bis 10 bar und der Wassergehalt des eingesetzten Diesters der Formel I maximal 100 ppm bezogen auf das Gewicht beträgt.

2. Verfahren nach Anspruch 1, **dadurch gekennzeichnet, dass** zumindest zeitweise eine Lagertemperatur von 40 °C bis 130 °C, bevorzugt von maximal 20 °C über dem Schmelzpunkt des Diesters der Formel I eingehalten wird.

3. Verfahren nach einem der vorhergehenden Ansprüche, **dadurch gekennzeichnet, dass** der Diester unmittelbar aus dem Herstellungsprozess bei einer Temperatur von 90 °C bis 160 °C in flüssigem Aggregatszustand entnommen und in das Lagergefäß überführt wird.

4. Verfahren nach einem der vorhergehenden Ansprüche, **dadurch gekennzeichnet, dass** die Lagerung und/oder der Transport bei einem Druck von 1 bar bis 5 bar unter Inertgas erfolgt.

5. Verfahren nach einem der vorhergehenden Ansprüche, **dadurch gekennzeichnet, dass** die Inertisierung des Lagergefäßes durch Spülen mit mindestens einem im Wesentlichen von Wasser- und Sauerstoff freien Gas, bevorzugt einem gegenüber dem Diester der allgemeinen Formel I chemisch inerten Gas, besonders bevorzugt Stickstoff und/oder Argon, erfolgt.

6. Verfahren nach einem der vorhergehenden Ansprüche, **dadurch gekennzeichnet, dass** als Diester der allgemeinen Formel I Dilactid eingesetzt wird.

7. Verfahren nach vorhergehendem Anspruch, **dadurch gekennzeichnet, dass** das Dilactid durch die folgenden Verfahrensschritte hergestellt wird:
a) Polykondensation von Milchsäure zu einem Präpolymer,
b) cyclisierende Depolymerisation und
c) Aufreinigung des Dilactids.

8. Verfahren nach einem der beiden vorhergehenden Ansprüche, **dadurch gekennzeichnet, dass** L,L-Dilactid, D,D-Dilactid, meso-Dilactid und/oder Mischungen hieraus gelagert und/oder transportiert werden.

9. Verfahren nach einem der vorhergehenden Ansprüche, **dadurch gekennzeichnet, dass** die im Wesentlichen von Verunreinigungen durch zum Diester der Formel I korrespondierende alpha-Hydroxycarbonsäure der Formel II 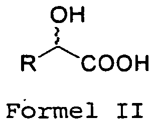 und/oder korrespondierenden Oligomeren der alpha-Hydroxycarbonsäure der Formel III, 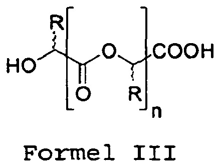 wobei n = 1 bis 10 ist und R je wie oben stehend definiert ist, herrührende Carboxylendgruppen-konzentration des eingesetzten Diesters der Formel I maximal 20 mmol/kg, bevorzugt maximal 10 mmol/kg, besonders bevorzugt maximal 5 mmol/kg, insbesondere maximal 2 mmol/kg beträgt.

10. Verfahren nach einem der vorhergehenden Ansprüche, **dadurch gekennzeichnet, dass** der Wassergehalt des eingesetzten Diesters der Formel I bezogen auf das Gewicht, maximal 50 ppm, bevorzugt maximal 20 ppm beträgt.

## Claims

1. Method for storing and/or transporting a cyclic diester of the
general formula I 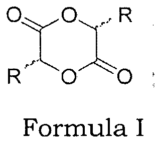 R being selected from hydrogen or linear or branched aliphatic radicals with 1 to 6 carbon atoms, **characterised in that** the diester of the general formula I is transferred, after production, into a storage vessel, rendered inert, without interruption and directly in the liquid aggregate state and is stored in the storage vessel and/or transported at least at times at 40 to 150°C, the storage and/or the transport being at a pressure of 0.1 bar to 10 bar and the water content of the diester which is used being max. 100 ppm, relative to the weight.

2. Method according to claim 1, **characterised in that** a storage temperature of 40°C to 130°C, preferably of at most 20°C above the melting point of the diester of formula I, is maintained at least at times.

3. Method according to one of the preceding claims, **characterised in that** the diester is removed in the liquid aggregate state directly from the production process at a temperature of 90°C to 160°C and is transferred into the storage vessel.

4. Method according to one of the preceding claims, **characterised in that** the storage and/or the transport is effected at a pressure of 1 bar to 5 bar under inert gas.

5. Method according to one of the preceding claims, **characterised in that** the storage vessel is rendered inert by purging with at least one gas which is essentially free of water and oxygen, preferably a gas chemically inert relative to the diester of the general formula I, particularly preferred nitrogen and/or argon.

6. Method according to one of the preceding claims, **characterised in that** dilactide is used as diester of the general formula I.

7. Method according to the preceding claim, **characterised in that** the dilactide is produced by the following method steps:
a) polycondensation of lactic acid to form a prepolymer,
b) cyclicising depolymerisation and
c) purification of the dilactide.

8. Method according to one of the two preceding claims, **characterised in that** L,L-dilactide, D,D-dilactide, meso-dilactide and/or mixtures hereof are stored and/or transported.

9. Method according to one of the preceding claims, **characterised in that** the carboxyl end group concentration of the diester of formula I which is used, resulting essentially from impurities due to alpha-hydroxycarboxylic acid, corresponding to the diester of formula I, of formula II 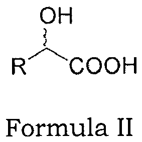 and/or from corresponding oligomers of the alphahydroxycarboxylic acid of formula III, 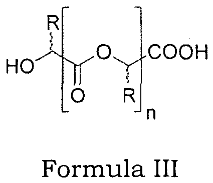 n = 1 to 10 and R being defined respectively as above, is at most 20 mmol/kg, preferably at most 10 mmol/kg, particularly preferred at most 5 mmol/kg, in particular at most 2 mmol/kg.

10. Method according to one of the preceding claims, **characterised in that** the water content of the diester of formula I which is used, relative to the weight, is at most 50 ppm, preferably at most 20 ppm.

## Revendications

1. Procédé de stockage et/ou de transport d'un diester cyclique de formule
générale I 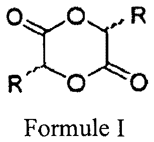 R étant choisi parmi l'hydrogène ou les radicaux aliphatiques à chaîne droite ou ramifiée possédant de 1 à 6 atomes de carbone, **caractérisé en ce que** le diester de formule générale I est, après sa préparation, transvasé directement sans interruption, à l'état liquide, dans un récipient de stockage inertisé, et est stocké et/ou transporté au moins par intermittence dans le récipient de stockage de 40 à 150 °C, le stockage et/ou le transport étant effectués sous une pression de 0,1 à 10 bar, et la teneur en eau du diester utilisé de formule I étant au maximum de 100 ppm en poids.

2. Procédé selon la revendication 1, **caractérisé en ce qu'**au moins par intermittence une température de stockage de 40 à 130 °C est maintenue, de préférence à maximum 20 °C au-dessus du point de fusion du diester de formule I.

3. Procédé selon l'une quelconque des revendications précédentes, **caractérisé en ce que** le diester est prélevé immédiatement du procédé de préparation, à une température de 90 à 160 °C à l'état liquide, et transvasé dans le récipient de stockage.

4. Procédé selon l'une quelconque des revendications précédentes, **caractérisé en ce que** le stockage et/ou le transport sont effectués sous une pression de 1 à 5 bar sous gaz inerte.

5. Procédé selon l'une quelconque des revendications précédentes, **caractérisé en ce que** l'inertisation du récipient de stockage est réalisée par rinçage avec au moins un gaz essentiellement exempt d'hydrogène et d'oxygène, de préférence un gaz chimiquement inerte vis-à-vis du diester de formule générale I, d'une manière particulièrement préférée l'azote et/ou l'argon.

6. Procédé selon l'une quelconque des revendications précédentes, **caractérisé en ce qu'**on utilise en tant que diester de formule générale I un dilactide.

7. Procédé selon la revendication précédente, **caractérisé en ce que** le dilactide est préparé par les étapes de procédé suivantes :
a) polycondensation d'acide lactique pour donner un prépolymère,
b) dépolymérisation avec cyclisation, et
c) purification du dilactide.

8. Procédé selon l'une quelconque des deux revendications précédentes, **caractérisé en ce que** le L,L-dilactide, le D,D-dilactide, le méso-dilactide et/ou leurs mélanges sont stockés et/ou transportés.

9. Procédé selon l'une des revendications précédentes, **caractérisé en ce que** la concentration des groupes terminaux carboxyle, du diester utilisé de formule I, provenant essentiellement d'impuretés dues à un acide alpha-hydroxycarboxylique de formule II, correspondant au diester de formule I 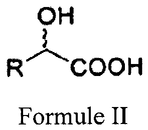 et/ou à des oligomères correspondants de l'acide alpha-hydroxycarboxylique, de formule III 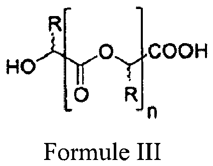 n = 1 à 10 et chaque radical R étant tel que défini ci-dessus, est au maximum de 20 mmol/kg, de préférence au maximum de 10 mmol/kg, d'une manière particulièrement préférée au maximum de 5 mmol/kg, en particulier au maximum de 2 mmol/kg.

10. Procédé selon l'une quelconque des revendications précédentes, **caractérisé en ce que** la teneur en eau du diester utilisé de formule I est, rapporté en poids, au maximum de 50 ppm, de préférence au maximum de 20 ppm.
